# EUROPEAN PATENT APPLICATION

(11) **EP 4 130 036 A1**
(43) Date of publication of application: **08.02.2023**
(21) Application number: 21779835.4
(22) Date of filing: 16.03.2021
(51) Int. Cl.: C07K 16/18, A61K 39/395, A61K 47/65, A61K 47/68, A61P 35/00, C07K 5/10, C12N 15/13

(54) **ANTIBODY DRUG CONJUGATE**

(30) Priority: 30.03.2020 JP 2020060968
(71) Applicant: National Cancer Center, Tokyo 104-0045 (JP); RIN Institute Inc., Tokyo, 104-0045 (JP)
(72) Inventor: MATSUMURA, Yasuhiro, Kashiwa-shi, Chiba 277-8577 (JP); TSUMURA, Ryo, Kashiwa-shi, Chiba 277-8577 (JP)
(74) Representative: J A Kemp LLP
(86) International application number: PCT/JP2021/010689
(87) International publication number: WO 2021/200131

(57) **Abstract**

The present invention relates to an antibody drug conjugate in which an antibody that binds to tissue factor and Dxd that is the cytotoxic agent are bound via a linker composed of maleimide caproyl-glycine-glycine-phenylalanine-glycine.

## Description

### [Technical Field]

The present invention relates to an antibody drug conjugate and an anti-cancer composition containing the same.

### [Background Art]

Tissue factor (TF) is expressed in many human cancer cells, and clinical studies have demonstrated a positive correlation between expression and malignancy. In addition, the present inventors have produced an antibody targeting tissue factor and an antibody drug conjugate (ADC) containing an anti-tissue factor antibody and a cytotoxic agent (PTLs 1 and 2, and NPL 1).

### [Citation List]

### [Patent Literature]

[PTL 1] International Publication No. WO2015/115656
[PTL 2] International Publication No. WO2019/087994
[PTL 3] Japanese Unexamined Patent Application Publication No. 2018/188455

### [Non Patent Literature]

[NPL 1] Koga Y. et al., Int J Cancer, September 15, 2015, Vol. 137, Issue 6, pp. 1457 to 1466

### [Summary of Invention]

### [Technical Problem]

An object of the present invention is to provide an antibody drug conjugate containing an anti-tissue factor antibody and a cytotoxic agent, which exerts an antitumor effect in vivo.

### [Solution to Problem]

The present inventors conducted intensive studies to achieve the above object, and prepared an ADC in which the cytotoxic agent monomethyl auristatin E (MMAE) was bound to an anti-tissue factor antibody via a linker composed of maleimide-polyethylene glycol 12-valine-citrulline-p-aminobenzyl alcohol (hereinafter also referred to as "hu1084-PEG12-MMAE").

The present inventors further prepared an ADC in which the cytotoxic agent MMAE was bound to an anti-tissue factor antibody via a linker composed of maleimide caproyl-valine-citrulline-p-aminobenzyl alcohol (hereinafter also referred to as "hu1084-MMAE").

The present inventors also prepared an ADC in which the cytotoxic agent Dxd was bound to an anti-tissue factor antibody via a linker composed of maleimide caproyl-glycine-glycine-phenylalanine-glycine (hereinafter also referred to as "hu1084-Dxd") (for the linker and Dxd, see PTL 3).

Then, the cell-killing effect of these ADCs on human pancreatic adenocarcinoma cell lines (BxPC-3, PSN-1, HPAF-II) was analyzed, and as a result, in all human pancreatic adenocarcinoma cells, hu1084-PEG12-MMAE or hu1084-MMAE showed a higher cell-killing effect than hu1084-Dxd. In particular, hu1084-Dxd showed no cell-killing effect on PSN-1.

Thus, it was suggested that ADC containing anti-tissue factor antibody and MMAE was more effective against human pancreatic cancer. However, as a result of evaluation using a nude mouse (PDX model) transplanted with human pancreatic cancer tissue, it was found that, surprisingly, hu1084-Dxd showed a significant tumor growth inhibitory effect on human-derived pancreatic cancer tissue in vivo, contrary to the in vitro results, and tumor regression was also observed. Thus, the present invention has been completed.

That is, the present invention relates to an ADC in which Dxd is bound to an anti-tissue factor antibody via a linker composed of maleimide caproyl-glycine-glycine-phenylalanine-glycine, and more specifically provides the following.
<1> A conjugate in which an antibody that binds to tissue factor is bound to a linker and drug represented by the following formula. 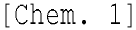 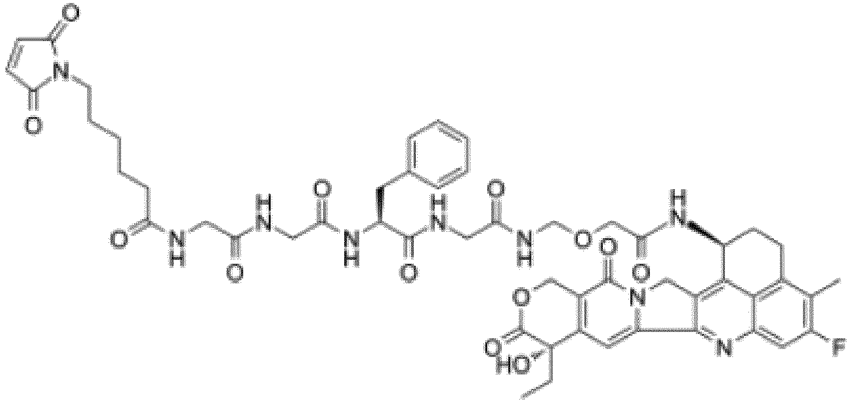
<2> The conjugate according to <1>, wherein the antibody that binds to tissue factor has a dissociation rate constant kd of 1 × 10⁻⁴ (1/s) or more and an association rate constant Ka of 1 × 10⁴ (1/Ms) or more.
<3> The conjugate according to <1> or <2>, wherein the antibody that binds to tissue factor is an antibody that comprises
   a heavy chain variable region containing, as CDRs 1 to 3, the amino acid sequences of SEQ ID NOs: 1 to 3, or the amino acid sequences of SEQ ID NOs: 1 to 3 in at least any one of which one or more amino acids are substituted, deleted, added, and/or inserted and
   a light chain variable region containing as CDRs 1 to 3, the amino acid sequences of SEQ ID NOs: 5 to 7, or the amino acid sequences of SEQ ID NOs: 5 to 7 in at least any one of which one or more amino acids are substituted, deleted, added, and/or inserted.
<4> The conjugate according to <1> or <2>, wherein the antibody that binds to tissue factor is an antibody that comprises
   a heavy chain variable region containing the amino acid sequence of SEQ ID NO: 4, the amino acid sequence having a homology of 80% or more with the amino acid sequence of SEQ ID NO: 4, or the amino acid sequence of SEQ ID NO: 4 in at least any portion of which one or more amino acids are substituted, deleted, added, and/or inserted and
   a light chain variable region containing the amino acid sequence of SEQ ID NO: 8, the amino acid sequence having a homology of 80% or more with the amino acid sequence of SEQ ID NO: 8, or the amino acid sequence of SEQ ID NO: 8 in at least any portion of which one or more amino acids are substituted, deleted, added, and/or inserted.
<5> The conjugate according to any one of <1> to <4>, wherein an average number of linkers and drugs bonded per antibody is 3 to 8.
<6> An anti-cancer composition comprising: the conjugate according to any one of <1> to <5>.
<7> An anti-pancreatic cancer composition comprising: the conjugate according to any one of <1> to <5>.

The present invention also relates to the use of the conjugate for the production of anti-cancer compositions (such as anti-pancreatic cancer compositions), the use of the conjugate for anti-cancer applications, and a method for treating cancer including administering an effective amount of the conjugate to a subject.

In the present invention, the "tissue factor (TF)" is a blood coagulation factor, and is a protein also called F3, CD142, TFA, coagulation factor III, thromboplastin, or tissue thromboplastin. Tissue factor is a transmembrane glycoprotein and is known as an initiation factor for extrinsic blood coagulation reactions. The tissue factor is preferably human tissue factor. The amino acid sequence of human tissue factor is, for example, as shown in NCBI Reference Sequence: NP_001984. However, the DNA sequence of a gene is mutated in nature (that is, non-artificially) due to the mutation or the like, and the amino acid sequence of the protein encoded by the mutation is also modified accordingly. Therefore, the "tissue factor" according to the present invention is not specified as a protein composed of the typical amino acid sequence, and includes such a naturally occurring variant.

The "association rate constant" (ka) and the "dissociation rate constant" (kd) are rate constants in the association and dissociation reactions between two molecules. Each can be determined, for example, by measurement by surface plasmon resonance (SPR), as shown in the Examples below. SPR measurement of the association between an antibody and an antigen is well known, and those skilled in the art can obtain the association rate constant ka and dissociation rate constant kd of the antibody based on the well-known technique. In SPR measurement, the association rate constant can be obtained from the amount of change in RU in the phase in which the analyte is allowed to flow at a constant concentration (association phase), and after that, the association rate constant can be obtained from the amount of change in RU in the phase in which the running buffer is allowed to flow (dissociation phase). The measurement can be performed by single-cycle kinetics. Further, the analysis can be performed by bivalent analysis. The curve fit of the approximate curve to the measured SPR sensorgram can be performed by the kinetic titration 1:1 interaction model. For details of the curve fit, see Karlsson, R., Katsamba, P. S., Nordin, H., Pol, E. and Myszka, D. G. (2006). "Analyzing a kinetic titration series using affinity biosensors.", Anal. Biochem. 349 (1): 136-47.

The antibody association rate constant ka and dissociation rate constant kd can also be determined according to the manufacturer's manual using, for example, an SPR device such as Biacore (registered trademark) commercially available from GE Healthcare. More specifically, the SPR measuring device is also equipped with a program for obtaining ka and kd, and ka and kd can be calculated from the SPR sensorgram. For example, the SPR sensorgram obtained with a Biacore (registered trademark) device is analyzed using Biacore T200 Evaluation Software, a bivalent analyte model is employed as the fitting model to derive the fitting curve, and ka, kd, and KD can also be calculated as the kinetics parameters of the antibody or ADC.

### [Advantageous Effects of Invention]

According to the present invention, it is possible to provide an antibody drug conjugate containing an anti-tissue factor antibody and a cytotoxic agent, which exerts an antitumor effect in vivo.

### [Brief Description of Drawings]

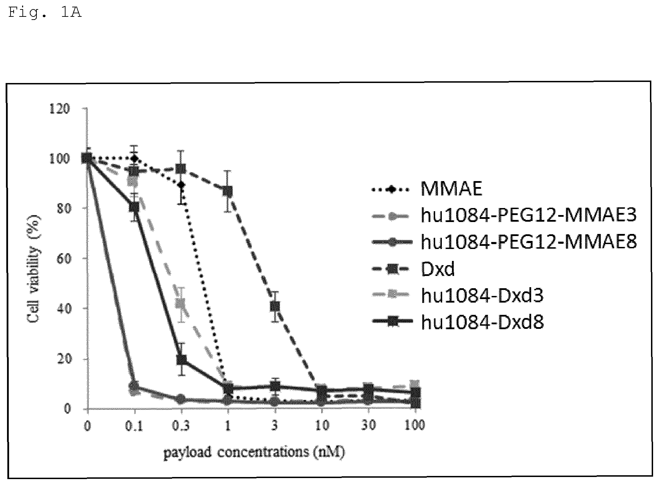

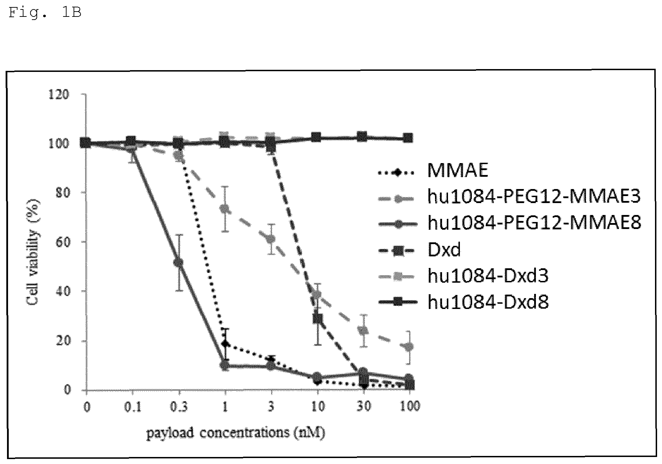

[Fig. 1A] Fig. 1A is a graph showing the results of analyzing the cell-killing effect of an antibody drug conjugate and a single drug on a human pancreatic adenocarcinoma cell line (BxPC-3). In the figure, the vertical axis indicates the survival rate of the human pancreatic adenocarcinoma cell line, and the horizontal axis indicates the concentration of the drug added to the cell line culture medium (in terms of each drug) (the notation of the vertical axis and horizontal axis of the graph is the same as well in Figs. 1B to 1E below).
[Fig. 1B] Fig. 1B is a graph showing the results of analyzing the cell-killing effect of an antibody-drug conjugate and a single drug on a human pancreatic adenocarcinoma cell line (PSN-1).

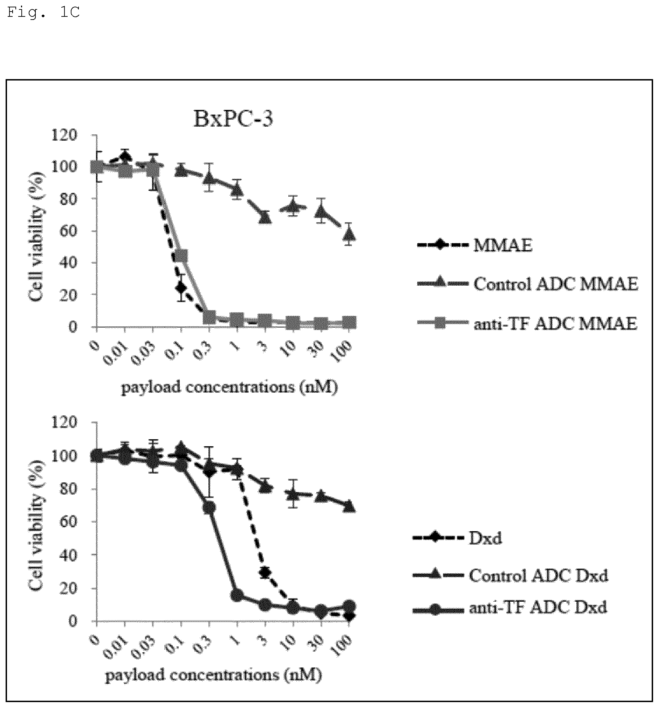

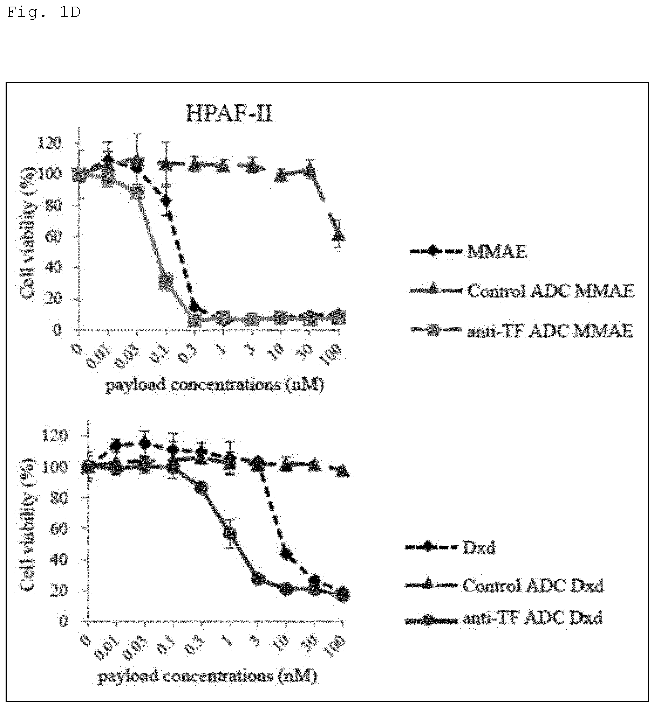

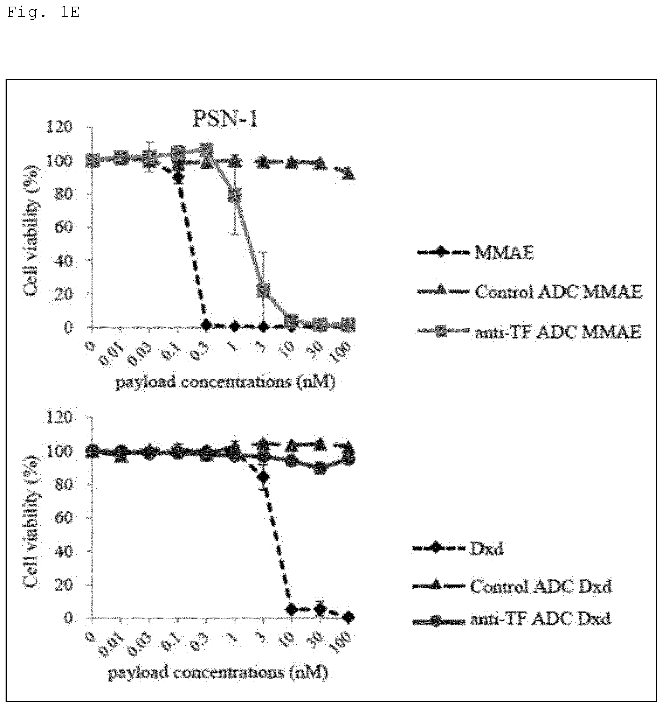

[Fig. 1C] Fig. 1C is a graph showing the results of analyzing the cell-killing effect of an antibody-drug conjugate and a single drug on a human pancreatic adenocarcinoma cell line (BxPC-3). In the figure, "anti-TF ADC MMAE" represents hu1084-MMAE, and "Control ADC MMAE" represents a modified amino acid in the CDR region of hu1084 in hu1084-MMAE, which no longer recognizes human tissue factor. The average number of the linkers and drugs bonded per antibody (DAR) in the antibody drug conjugate containing MMAE is 3 to 4. The "anti-TF ADC Dxd" represents hu1084-Dxd, and "Control ADC Dxd" represents a modified amino acid in the CDR region of hu1084 in hu1084-Dxd, which no longer recognizes human tissue factor. The DAR in these antibody drug conjugates containing Dxd is 7 to 8 (these notations are the same as well in Figs. 1D and 1E) .
[Fig. 1D] Fig. 1D is a graph showing the results of analyzing the cell-killing effect of an antibody drug conjugate and a single drug on a human pancreatic adenocarcinoma cell line (HPAF-II).
[Fig. 1E] Fig. 1E is a graph showing the results of analyzing the cell-killing effect of an antibody drug conjugate and a single drug on a human pancreatic adenocarcinoma cell line (PSN-1).

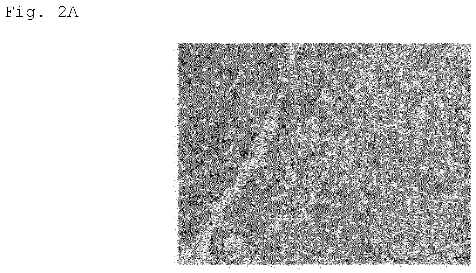

[Fig. 2A] Fig. 2A is a photomicrograph showing the results of analyzing tumor tissue formed in a nude mouse (no, 2) transplanted with human pancreatic cancer tissue (human pancreatic tumor tissue fragments, model number: PAN-02-JCK (Central Institute for Experimental Animals)) by immunostaining using an anti-tissue factor antibody. The scale bar in the figure represents 200 µm.

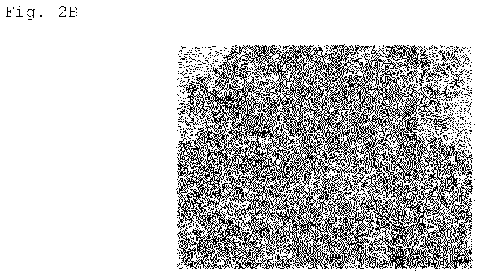

[Fig. 2B] Fig. 2B is a photomicrograph showing the results of analyzing tumor tissue formed in a nude mouse (no, 4) transplanted with human pancreatic cancer tissue (human pancreatic tumor tissue fragments, model number: PAN-04-JCK (Central Institute for Experimental Animals)) by immunostaining using an anti-tissue factor antibody. The scale bar in the figure represents 200 µm.

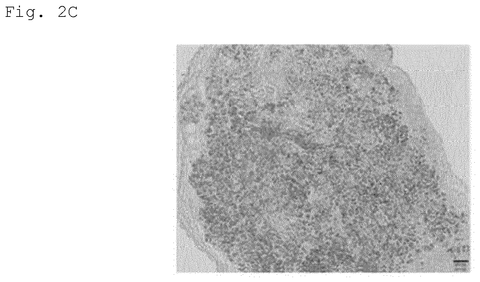

[Fig. 2C] Fig. 2C is a photomicrograph showing the results of analyzing tumor tissue formed in a nude mouse (no, 8) transplanted with human pancreatic cancer tissue (human pancreatic tumor tissue fragments, model number: PAN-08-JCK (Central Institute for Experimental Animals)) by immunostaining using an anti-tissue factor antibody. The scale bar in the figure represents 200 µm.

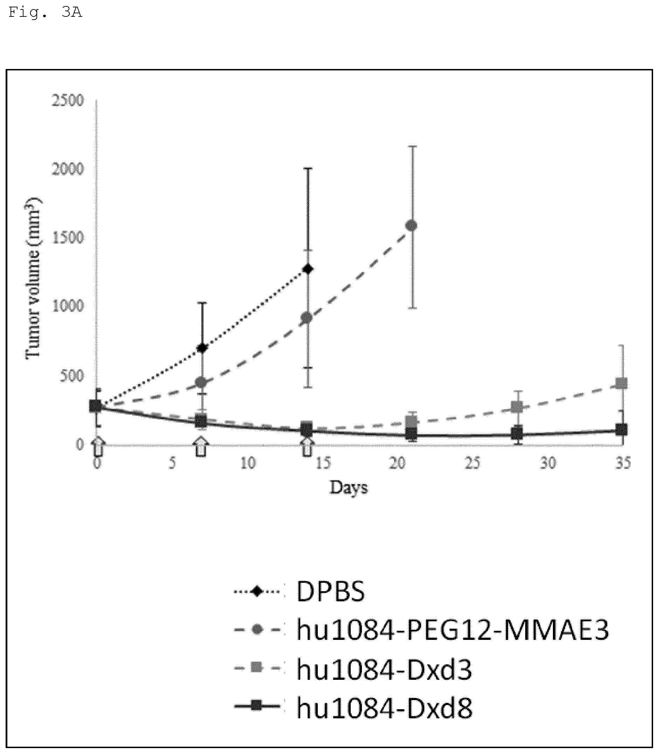

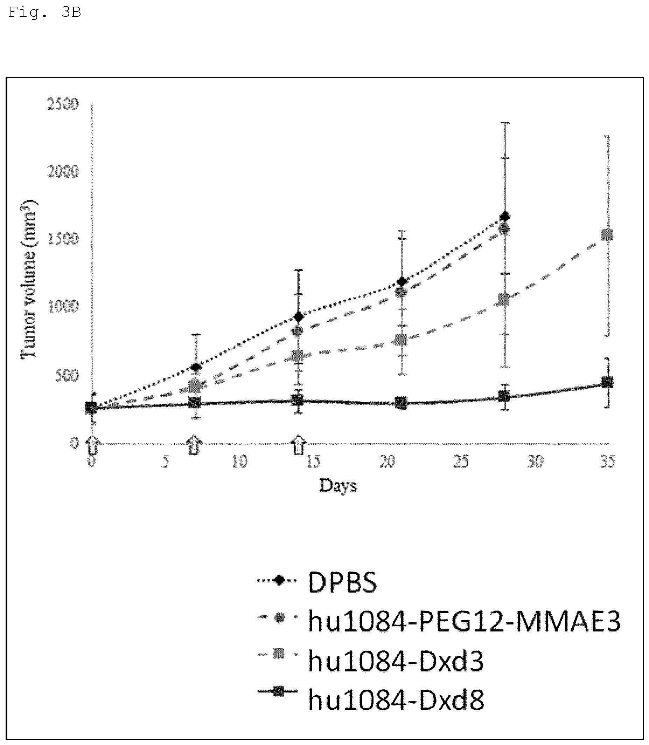

[Fig. 3A] Fig. 3A is a graph showing the results of analyzing the antitumor effect of an antibody drug conjugate in the nude mouse (no, 2). In the figure, the vertical axis indicates the tumor size, and the horizontal axis indicates the number of days since the start of administrating the antibody drug conjugate (the notation of the vertical axis and horizontal axis of the graph is the same as well in Figs. 3B to 3E below).
[Fig. 3B] Fig. 3B is a graph showing the results of analyzing the antitumor effect of an antibody drug conjugate in the nude mouse (no, 4). In the figure, the vertical axis indicates the tumor size, and the horizontal axis indicates the number of days since the start of administrating the antibody drug conjugate.

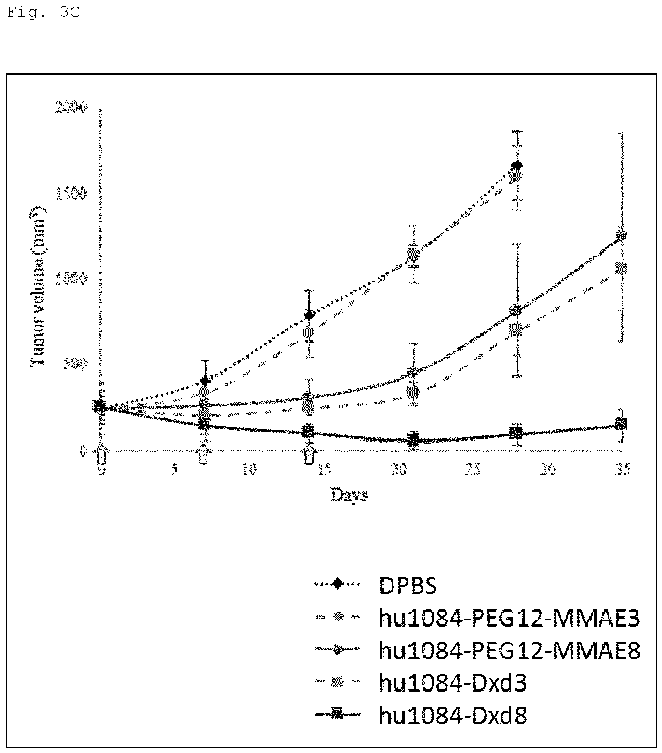

[Fig. 3C] Fig. 3C is a graph showing the results of analyzing the antitumor effect of an antibody drug conjugate in the nude mouse (no, 8). In the figure, the vertical axis indicates the tumor size, and the horizontal axis indicates the number of days since the start of administrating the antibody drug conjugate.

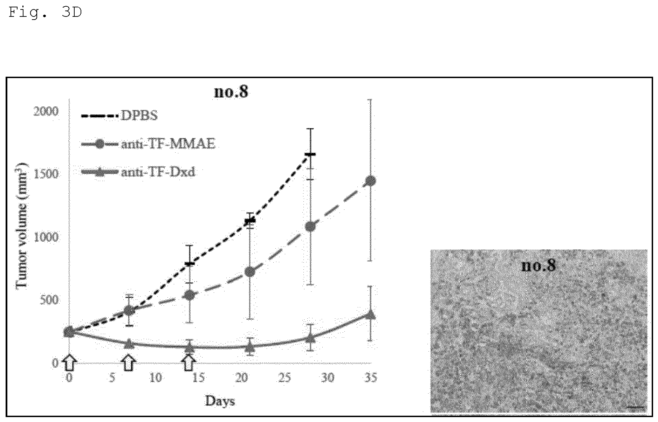

[Fig. 3D] Fig. 3D is a graph showing the results of analyzing the antitumor effect of an antibody drug conjugate in the nude mouse (no, 8). In the figure, "anti-TF-MMAE" and "anti-TF-Dxd" represent hu1084-MMAE (DAR: 3) and hu1084-Dxd (DAR: 8), respectively. The figure also shows the results of analyzing the tumor tissue formed in the nude mouse (no, 8) by immunostaining using an anti-tissue factor antibody. The scale bar in the micrograph represents 200 um.

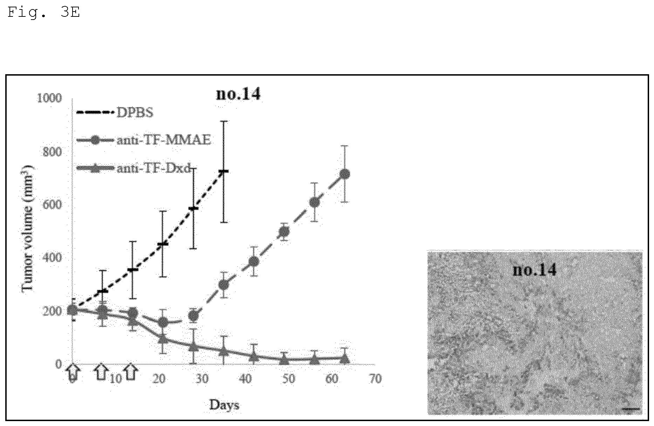

[Fig. 3E] Fig. 3E is a graph showing the results of analyzing the antitumor effect of an antibody drug conjugate in a nude mouse (no, 14) transplanted with human pancreatic cancer tissue (human pancreatic tumor tissue fragments, model number: PAN-14-JCK (Central Institute for Experimental Animals)). The figure also shows the results of analyzing the tumor tissue formed in the nude mouse (no, 14) by immunostaining using an anti-tissue factor antibody. The scale bar in the micrograph represents 200 um.

### [Description of Embodiments]

### <Antibody Drug Conjugate>

As shown in Examples described later, it has been clarified that a conjugate (antibody drug conjugate, ADC) in which an antibody that binds to tissue factor and the cytotoxic agent Dxd are bound to each other via a linker composed of maleimide caproyl-glycine-glycine-phenylalanine-glycine exhibits a significant tumor growth inhibitory effect on cancer in vivo, and further exhibits a tumor regression effect. Therefore, the present invention provides the antibody drug conjugate.

### (Antibody that binds to Tissue Factor)

The "antibody" in the present invention includes all classes of immunoglobulins (such as IgG, IgE, IgM, IgD, IgA, and IgY) and subclasses thereof (such as IgG1, IgG2, IgG3, IgG4, IgA1, and IgA2). The "antibody" includes polyclonal antibodies and monoclonal antibodies, and is meant to include the form of functional fragments of antibodies. A "polyclonal antibody" is an antibody preparation containing different antibodies against different epitopes. By "monoclonal antibody" is meant an antibody (including an antibody fragment) obtained from a substantially homogeneous population of antibodies. In contrast to polyclonal antibodies, monoclonal antibodies recognize a single determinant on an antigen. The antibody of the present invention is preferably a monoclonal antibody. The antibody of the invention is an antibody that has been separated and/or recovered (that is, isolated) from components of the natural environment.

Such an antibody can be prepared by the hybridoma method or by the recombinant DNA method.

Typical hybridoma methods include Kohler and Milstein method (Kohler & Milstein, Nature, 256: 495 (1975)). The antibody-producing cells used in the cell fusion step in this method are spleen cells, lymph node cells, peripheral blood leukocytes, and the like of animals (such as rats, mice, hamsters, rabbits, monkeys, and goats) immunized with an antigen (tissue factor, a partial peptide thereof, a protein with Fc or other protein fused with them, or cells expressing these). It is also possible to use antibody-producing cells obtained by allowing an antigen to act in a medium against the above-mentioned cells or lymphocytes isolated in advance from a non-immune animal. Various known cell lines can be used as myeloma cells. The antibody-producing cells and myeloma cells may be of different animal species origin as long as they can be fused, but are preferably of the same animal species origin. Hybridomas are produced, for example, by cell fusion between spleen cells obtained from antigen-immunized mice and mouse myeloma cells, and subsequent screening can give hybridomas that produce tissue factor-specific monoclonal antibodies. Monoclonal antibodies against tissue factor can be obtained by culturing hybridomas and from the ascites of mammals administered with hybridomas.

The recombinant DNA method is a method in which a DNA encoding the antibody of the present invention is cloned from a hybridoma, B cell, or the like and incorporated into an appropriate vector, which is introduced into a host cell (for example, mammalian cell lines such as CHO cells and HEK cells, Escherichia coli, yeast cells, insect cells, plant cells, and the like) to produce the antibody of the present invention as a recombinant antibody (for example, P. J. Delves, Antibody Production: Essential Techniques, 1997 WILEY, P. Shepherd and C. Dean Monoclonal Antibodies, 2000 OXFORD UNIVERSITY PRESS, Vandamme A. M. et al., Eur. J. Biochem. 192: 767-775 (1990)). In the expression of the DNA encoding the antibody of the present invention, a DNA encoding a heavy chain or light chain may be separately incorporated into expression vectors to transform a host cell, or a DNA encoding heavy and light chains may be integrated into a single expression vector to transform a host cell (see International Publication No. WO94/11523). The antibody of the present invention can be obtained in a substantially pure and homogeneous form by culturing the host cell, separating and purifying it in the host cell or from the culture solution. For the separation and purification of antibodies, the normal method used in the purification of polypeptides can be used. If a transgenic animal production technique is used to produce a transgenic animal (such as a cow, goat, sheep, or pig) incorporated with an antibody gene, it is also possible to obtain a large amount of monoclonal antibody derived from an antibody gene from the milk of that transgenic animal.

The antibody of the present invention may bind to tissue factor, and its origin, type, shape, and the like are not particularly limited. Specific examples include non-human animal-derived antibodies (such as rat antibodies, mouse antibodies, and camel antibodies), human-derived antibodies, chimeric antibodies, humanized antibodies, and functional fragments of these antibodies. When the antibody of the present invention is administered to humans as a pharmaceutical, a chimeric antibody or a humanized antibody is desirable from the viewpoint of reducing side effects.

In the present invention, the "chimeric antibody" is an antibody in which the variable region of an antibody of a certain species and the constant region of an antibody of a different species are linked. A chimeric antibody can be obtained, for example, by cutting out an antibody variable portion (variable region) that binds to an antigen from the gene of the monoclonal antibody produced as described above, binding it to the antibody constant portion (constant region) gene derived from human bone marrow, and incorporating it into an expression vector followed by introduction into a host for production (for example, Japanese Unexamined Patent Application Publication No. Hei 8-280387, US Patent No. 4816397, US Patent No. 4816567, and US Patent No. 5807715).

As the constant region of a chimeric antibody, a human-derived antibody is usually used. For example, in a heavy chain, Cγ1, Cγ2, Cγ3, Cγ4, Cµ, Cδ, Cα1, Cα2, and Cε can be used as constant regions. Further, in a light chain, Cκ and Cλ can be used as a constant region. The amino acid sequences of these constant regions and the base sequences encoding them are known. Also, in order to improve the stability of an antibody itself or the stability of antibody production, one or several amino acids in the constant region of a human-derived antibody may be substituted, deleted, added, and/or inserted.

In the present invention, the "humanized antibody" is an antibody obtained by transplanting (CDR grafting) the gene sequence of the antigen binding site (CDR) of an antibody derived from a non-human (such as a rat) into a human-derived antibody gene, and the production method thereof is known such as overlap extension PCR (such as European Patent Application Publication No. 239400, European Patent Application Publication No. 125023, International Publication No. WO90/07861, International Publication No. WO96/02576). The variable region of an antibody is usually composed of three CDRs sandwiched between four framework regions (FRs). The CDR is substantially the region that determines the binding specificity of the antibody. While the amino acid sequences of CDRs are highly diverse, the amino acid sequences making up FRs often show high homology even among antibodies with different binding specificities. Therefore, it is generally said that the binding specificity of one antibody can be transplanted to another antibody by transplanting CDR. Further, from the viewpoint of maintaining the functions of a CDR, in the transplantation of a non-human-derived CDR into a human FR, a human FR is selected that has high homology with the FR derived from that non-human animal. Specifically, the amino acids in a CDR not only recognize antigens, but also coordinate with the amino acids of FR in the vicinity of the CDR and are involved in the maintenance of the loop structure of the CDR, so that it is preferable to use a human FR that has an amino acid sequence having high homology with the amino acid sequence of the FR adjacent to the CDR to be transplanted.

A search for known human FRs having high homology with non-human animal-derived FRs can be performed, for example, by using a search system specialized for antibodies available on the Internet (http://www.bioinf.org.uk/abysis/). Mutations can be introduced into non-CDR sequences of non-human-derived antibodies to match the sequences of human FRs thus obtained. Alternatively, if a gene (cDNA) encoding the amino acid sequence of human FR obtained by search is available, a non-human-derived CDR may be introduced into the sequence. The introduction of mutations and the like can be carried out using techniques known in the art such as nucleic acid synthesis and site-directed mutagenesis.

By qualitatively or quantitatively measuring and evaluating the antigen-binding activity of the humanized antibody thus produced, the FR of such a human-derived antibody can be suitably selected that when linked via a CDR, the CDR forms a good antigen binding site. Further, if necessary, according to the method described in Sato, K. et al., Cancer Res, 1993, 53, 851-856 and the like, the amino acid residue of FR can be substituted so that the CDR of the humanized antibody forms an appropriate antigen binding site. Further, by measuring and evaluating the antigen binding activity of the mutant antibody substituted with the amino acid, a mutant FR sequence having desired properties can be selected.

In the present invention, the "functional fragment" of an antibody is also referred to as an antigen-binding fragment, and means a part (partial fragment) of an antibody that specifically recognizes tissue factor. Specific examples thereof include Fab, Fab', F(ab')2, variable region fragment (Fv), disulfide bond Fv, single chain Fv (scFv), sc(Fv)2, diabodies, multispecific antibodies, and polymers thereof.

Here, "Fab" means a monovalent antigen binding fragment of an immunoglobulin composed of one light chain and a part of a heavy chain. It can be obtained by papain digestion of an antibody or by the recombinant method. "Fab'" differs from Fab by the addition of a few residues at the carboxy terminus of the heavy chain CH1 domain, including one or more cysteines in the hinge region of the antibody. By "F(ab')2" is meant a divalent antigen binding fragment of an immunoglobulin composed of both light chain and both heavy chain moieties.

A "variable region fragment (Fv)" is the smallest antibody fragment with complete antigen recognition and binding site. Fv is a dimer in which heavy chain variable regions and light chain variable regions are strongly linked by non-covalent bonds. A "single chain Fv (scFv)" includes a heavy chain variable region and light chain variable region of an antibody, which are present in a single polypeptide chain. "Sc(Fv)2" is a single chain obtained by binding two heavy chain variable regions and two light chain variable regions with a linker or the like. A "diabody" is a small antibody fragment with two antigen binding sites, where this fragment containing a heavy chain variable region bound to a light chain variable region within the same polypeptide chain, each region pairing with a complementary region of another chain. A "multispecific antibody" is a monoclonal antibody that has binding specificity for at least two different antigens. For example, two heavy chains can be prepared by co-expression of two immunoglobulin heavy chain/light chain pairs with different specificities.

The antibody of the present invention includes an antibody whose amino acid sequence is modified without reducing a desired activity (such as antigen binding activity). Such amino acid sequence variants can be produced, for example, by introducing a mutation into a DNA encoding the antibody chain of the humanized 1084 antibody described below, or by peptide synthesis. Examples of such modifications include substitutions, deletions, additions, and/or insertions of residues within the amino acid sequence of the antibody. The site where the amino acid sequence of the antibody is modified may be a constant region of the heavy or light chain of the antibody, or it may be a variable region (FR and CDR), as long as it has the same activity as the antibody before modification. Non-CDR amino acid modification is considered to have a relatively small effect on the antigen binding activity, but at present, a method is known that modifies the amino acid of a CDR to screen an antibody having an enhanced antigen-binding activity (PNAS, 102: 8466-8471 (2005), Protein Engineering, Design & Selection, 21: 485-493 (2008), International Publication No. WO2002/051870, J. Biol. Chem., 280: 24880-24887 (2005), Protein Engineering, Design & Selection, 21: 345-351 (2008), MAbs. Mar-Apr; 6(2): 437-45 (2014)). Further, at present, it is also possible to model an antibody having enhanced antigen-binding activity by using an integrated computational chemistry system or the like (such as Molecular Operating Environment, manufactured by CCG, Canada) (see, for example, http://www.rsi.co.jp/kagaku/cs/ccg/products/application/ protein.html). Further, as described in Protein Eng Des Sel. 2010 Aug; 23(8): 643-51, there are known examples in which CDR1 of the heavy chain variable region and CDR3 of the light chain variable region are not involved in the antigen-binding activity. Similarly, Molecular Immunology 44: 1075-1084 (2007) reports that in most antibodies, CDR2 of the light chain variable region is not involved in antigen-binding activity. As described above, in the antigen-binding activity of an antibody, the same activity can be exhibited without requiring all of CDRs 1 to 3 of each of the heavy chain variable region and light chain variable region. In fact, it has been reported that in Biochem Biophys Res Commun. 2003 Jul 18; 307 (1): 198-205, J Mol Biol. 2004 Jul 9; 340 (3): 525-42, J Mol Biol. 2003 Aug 29; 331 (5): 1109-20, antigen-binding activity is maintained by comprising at least one CDR of the original antibody. Thus, those skilled in the art can modify not only FR but also the amino acid sequence of CDR for the purpose of maintaining and improving the antigen-binding activity of the antibody.

As for the antibody of the present invention, the number of modified amino acids is preferably 10 amino acids or less (for example, 9 amino acids or less, 8 amino acids or less, 7 amino acids or less, 6 amino acids or less), more preferably 5 amino acids or less (for example, 4 amino acids or less), and further preferably 3 amino acids or less (for example, 2 amino acids or less, 1 amino acid). Amino acid modifications are preferably conservative substitutions. "Conservative substitution" means substituting with another amino acid residue having a chemically similar side chain. Groups of amino acid residues having chemically similar amino acid side chains are well known in the art to which the present invention belongs. For example, acidic amino acids (aspartic acid and glutamic acid), basic amino acids (lysine, arginine, and histidine), and neutral amino acids can be classified in terms of amino acids having hydrocarbon chains (glycine, alanine, valine, leucine, isoleucine, and proline), amino acids having hydroxy groups (serine and threonine), amino acids containing sulfur (cysteine and methionine), amino acids having amide groups (asparagine and glutamine), amino acids having imino groups (proline), and amino acids having aromatic groups (phenylalanine, tyrosine, tryptophan).

Further, an antibody having an antibody chain in which the modified amino acid sequence is composed of an amino acid sequence having 80% or more homology at the amino acid sequence level with the antibody chain of the humanized 1084 antibody described later is also included in the antibody of the present invention as long as it has the same activity as the antibody before modification. The homology may be at least 80%, preferably 85% or more, more preferably 90% or more, and further preferably 95% or more (for example, 96% or more, 97% or more, 98% or more, and 99% or more). In addition, sequence homology can be determined using a BLASTP (amino acid level) program (Altschul et al. J. Mol. Biol., 215: 403-410, 1990). The program is based on the algorithm BLAST by Karlin and Altschul (Proc. Natl. Acad. Sci. USA, 87: 2264-2268, 1990, Proc. Natl. Acad. Sci. USA, 90: 5873-5877, 1993). When analyzing the amino acid sequence by BLASTP, the parameters are, for example, score = 50 and wordlength = 3. In addition, when analyzing an amino acid sequence using the Gapped BLAST program, it can be performed as described in Altschul et al (Nucleic Acids Res. 25: 3389-3402, 1997). When using BLAST and Gapped BLAST programs, the default parameters of each program are used. Specific methods of these analysis methods are known. Further, "having the same activity" means that antigen-binding activity is equivalent (for example, 70% or more, preferably 80% or more, and more preferably 90% or more) to that of the target antibody (typically, humanized 1084 antibody).

Further, the modification of the antibody of the present invention may be a modification of the post-translational process of the antibody, for example, by changing the number or position of glycosylation sites. This makes it possible to, for example, improve ADCC activity of the antibody. Glycosylation of an antibody is typically N-linked or O-linked. Glycosylation of an antibody is highly dependent on the host cell used to express the antibody. The modification of glycosylation patterns can be performed by a known method such as introduction or deletion of a specific enzyme involved in sugar production (Japanese Unexamined Patent Application Publication No. 2008-113663, US Patent No. 5047335, US Patent No. 5510261, US Patent No. 5278299, and International Publication No. WO99/54342). Further, in the present invention, the amino acid to be deamidated or the amino acid adjacent to the amino acid to be deamidated may be substituted with another amino acid for the purpose of increasing the stability of the antibody to suppress the deamidation. Glutamic acid can also be substituted with another amino acid to increase antibody stability. The present invention also provides an antibody thus stabilized.

Further, the antibody of the present invention may have an internalizing ability (see PTL 1). "Internalization" means a phenomenon in which an antibody forms an immune complex with an antigen on the cell surface and then is taken up into a cell. Whether or not an antibody has an internalizing ability can be determined, for example, by a method in which an antibody bound with a labeling substance is brought into contact with a cell expressing tissue factor on the surface to confirm whether or not the labeling substance is transferred into the cell, a method of confirming whether or not cell death or inhibition of cell proliferation is induced when an antibody bound with a cytotoxic substance is brought into contact with a cell expressing tissue factor on the surface, and the like.

The antibody of the invention may also have at least one cytotoxic activity selected from ADCC activity and CDC activity. "ADCC activity (antibody-dependent cellular cytotoxicity)" means such an activity that when an antibody binds to the cell surface antigen of a target cell, effector cells (immune cells such as NK cells and monocytes) are further bound to the Fc region of that antibody to activate the cells, and accordingly, factors are released to kill the target cells. On the other hand, "CDC activity (complement-dependent cytotoxicity)" means the activity of lysing the target cells as a result of activating the complement system when the antibody binds to the target cells.

From the viewpoint that once associated to tissue factor and then rapidly dissociated, the ability to penetrate into solid tumors is high and the antitumor effect tends to be high as ADC (see PTL 2), the dissociation rate constant kd of the antibody according to the present invention is preferably 1 × 10⁻⁴ (1/s) or more, more preferably 2 × 10⁻⁴ (1/s) or more, further preferably 3 × 10⁻⁴ (1/s) or more, more preferably 4 × 10⁻⁴ (1/s) or more, further preferably 5 × 10⁻⁴ (1/s) or more, more preferably 6 × 10⁻⁴ (1/s) or more, further preferably 7 × 10⁻⁴ (1/s) or more, more preferably 8 × 10⁻⁴ (1/s) or more, further preferably 9 × 10⁻⁴ (1/s) or more, more preferably 10 × 10⁻⁴ (1/s) or more, and further preferably 15 × 10⁻⁴ (1/s) or more. In addition, from the same viewpoint, the association rate constant ka is 1 × 10⁴ (1/Ms) or more, more preferably 2 × 10⁴ (1/Ms) or more, further preferably 3 × 10⁴ (1/Ms) or more, more preferably 4 × 10⁴ (1/Ms) or more, further preferably 5 × 10⁴ (1/Ms) or more, and more preferably 6 × 10⁴ (1/Ms) or more. Further, the combination of kd and ka is, respectively, preferably 1 × 10⁻⁴ (1/s) or more and 1 × 10⁴ (1/Ms) or more, more preferably 3 × 10⁻⁴ (1/s) or more and 2 × 10⁴ (1/Ms) or more, further preferably 10 × 10⁻⁴ (1/s) or more and 3 × 10⁴ (1/Ms) or more, and more preferably 15 × 10⁻⁴ (1/s) or more and 6 × 10⁴ (1/Ms) or more. It should be noted that the upper limits of kd and ka can be those ranges of the antibody obtained by immunization. Also, the K_{D} value is preferably 1 to 100 nM, more preferably 10 to 50 nM, and further preferably 20 to 40 nM.

Examples of the antibody of the present invention include a monoclonal antibody produced from the rat-derived hybridoma clone No. 1084 described in PTL 2 (rat 1084 antibody). Further, as shown in Examples described later, an antibody obtained by humanizing an antibody derived from the rat (humanized 1084 antibody) is given as a more preferable example of the antibody of the present invention, and more specific examples include the following.

An antibody that binds to tissue factor that comprises
a heavy chain variable region containing, as CDRs 1 to 3, the amino acid sequences of SEQ ID NOs: 1 to 3, or the amino acid sequences of SEQ ID NOs: 1 to 3 in at least any one of which one or more amino acids are substituted, deleted, added, and/or inserted and
a light chain variable region containing, as CDRs 1 to 3, the amino acid sequences of SEQ ID NOs: 5 to 7, or the amino acid sequences of SEQ ID NOs: 5 to 7 in at least any one of which one or more amino acids are substituted, deleted, added, and/or inserted.

An antibody that binds to tissue factor that comprises
a heavy chain variable region containing the amino acid sequence of SEQ ID NO: 4, the amino acid sequence having a homology of 80% or more with the amino acid sequence of SEQ ID NO: 4, or the amino acid sequence of SEQ ID NO: 4 in at least any portion of which one or more amino acids are substituted, deleted, added, and/or inserted and
a light chain variable region containing the amino acid sequence of SEQ ID NO: 8, the amino acid sequence having a homology of 80% or more with the amino acid sequence of SEQ ID NO: 8, or the amino acid sequence of SEQ ID NO: 8 in at least any portion of which one or more amino acids are substituted, deleted, added, and/or inserted.

The homology and the number of amino acid modifications are as described above, and those skilled in the art can appropriately adjust the homology and the number of amino acid modifications while using an index such as the binding activity to tissue factor. Further, the site to be modified can be appropriately selected by those skilled in the art as described above, but is preferably a site in which the CDRs of the rat 1084 antibody and those of the humanized 1084 antibody have different amino acids, and more specifically at least one site (for example, 1, 2, 3, 4, 5, 6 or 7 sites) selected from positions 11 to 13, 15, and 16 from the N-terminal side of the heavy chain variable region CDR2, position 4 from the N-terminal side of the light chain variable region CDR1, and position 4 from the N-terminal side of the light chain variable region CDR2. Further, the amino acids at these sites may be any amino acid (so-called Xaa).

### (Linker and Drug)

In the ADC of the present invention, the above-mentioned antibody is bound to the linker and drug represented by the following formula (CAS number: 1599440-13-7) .

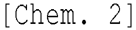

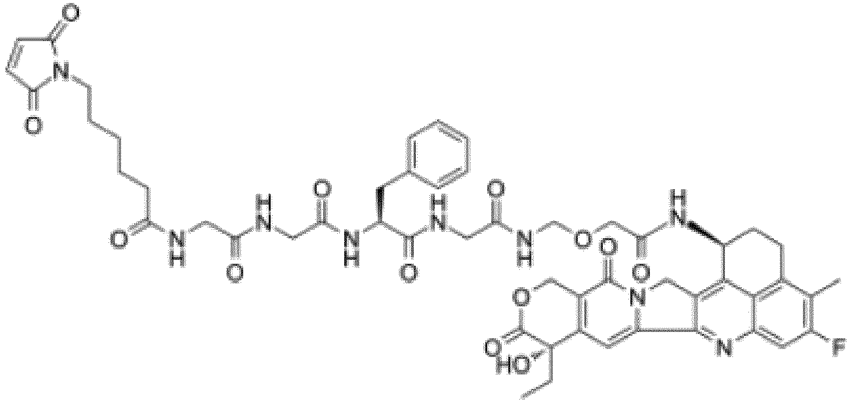

As described above, the linker according to the present invention is composed of maleimidocaproyl (MC)-glycine (Gly)-glycine (Gly)-phenylalanine (Phe)-glycine (Gly) . The drug is an antitumor compound (Dxd, CAS number: 1599440-33-1) represented by the following formula.

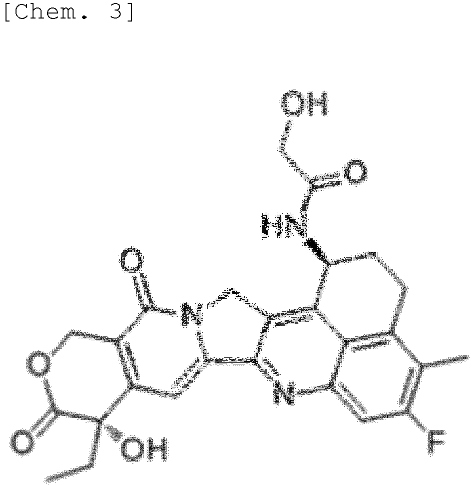

Note that since Dxd has a camptothecin structure, it is known that in an acidic aqueous medium (for example, about pH 3), the equilibrium is biased to the structure with a formed lactone ring (closed ring), while in a basic aqueous medium (for example, about pH 10), the equilibrium is biased to a structure with an open lactone ring (open ring). It is understood that even ADCs introduced with exatecan residues corresponding to such a closed ring structure and open ring structure are expected to have the same antitumor effect, and both of them are included in the scope of the present invention.

Such linkers and drugs can be appropriately synthesized by those skilled in the art using known methods. They can also be obtained by purchasing as a commercially available product (for example, product number: HY-13631E manufactured by MedChemExpress under the trade name: Deruxtecan).

The "bond" between the anti-tissue factor antibody according to the present invention and the above-mentioned linker and drug is, means a bond via a thioether group formed by a reaction between a thiol group (SH group, sulfhydryl group) in the antibody and a maleimide group at the linker terminal, as shown in the following formula. In the following formula, "mAb" represents an anti-tissue factor antibody according to the present invention, and "Dxd" represents a drug according to the present invention.

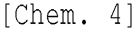

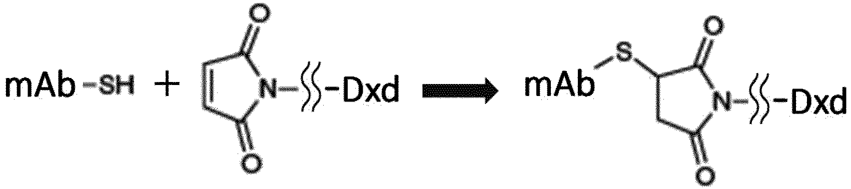

In the ADC of the present invention, the number of drug bonds per antibody molecule is determined in consideration of its efficacy and safety, specifying the reaction conditions such as the amount of raw materials and reagents to be reacted so that the number becomes a constant number. However, unlike the chemical reaction of small molecule compounds, it is usually obtained as a mixture of different numbers of drugs bound together. The number of drug bonds per antibody molecule is specified and expressed as an average value, that is, the average number of drug bonds. Therefore, unless otherwise specified in the present specification, the number of drug bonds means an average value.

As shown in Examples described later, the number of drug bonds per antibody molecule can be controlled by adjusting the type and concentration of the reducing agent used, the concentration of the linker and the drug based on the antibody (such as molar excess ratio), and the temperature or time in the binding reaction. The "reducing agent" is not particularly limited as long as it can reductively cleave the disulfide bonds in the antibody to generate bonds via the thiol groups, and examples thereof include dithiothreitol (DTT, DL-DTT), 2-mercaptoethylamine, mercaptoethanol, and TCEP.

In the present invention, the average number of drug bonds per antibody is preferably 3 to 8, more preferably 4 to 8, further preferably 5 to 8, more preferably 6 to 8, further preferably 7 to 8, and particularly preferably 8. The number of drug bonds per antibody molecule can be determined by those skilled in the art using a reagent such as DTNB for colorimetrically quantifying thiol groups, as shown in Examples described later.

Further, when left in the air or recrystallized, the ADC of the present invention may absorb water to be attached with adsorbed water or form a hydrate, and such water-containing compounds and salts are also included in the present invention.

One or more of the atoms constituting the ADC of the present invention may also include an unnatural proportion of atomic isotopes. Examples of atomic isotopes include ²H, ³H, ¹²⁵I, and ¹⁴C. Further, the ADC of the present invention may be bound with a labeling substance for detection in bioimaging techniques and the like. The labeling substance is appropriately selected according to the type of detection method to be used, and examples thereof include a radioactive labeling substance, a fluorescent labeling substance, a paramagnetic labeling substance, a superparamagnetic labeling substance, and an enzyme labeling substance. Further, the binding between the labeling substance and the ADC may be direct or indirect. Examples of the indirect binding include binding via a secondary antibody bound with a labeling substance or a polymer bound with a labeling substance (such as protein A or protein B).

More specifically, positron emitting nuclides can be used as the radioisotope to label the ADC. Antibodies can be labeled with positron emitting nuclides such as ⁶⁴Cu, ¹⁸F, ⁵⁵Co, ⁶⁶Ga, ⁶⁸Ga, ⁷⁶Br, ⁸⁹Zr, and ¹²⁴I. For labeling ADCs with these positron emitting nuclides, known methods can be used (Nucl Med Biol. 1999; 26 (8): 943-50., J Nucl Med. 2013; 54 (11): 1869-75.). In addition, after the ADC labeled with positron emitting nuclides is administered to the subject, the radiation emitted from the radionuclides is measured from outside the body by PET (positron emission tomography) and converted into an image by a computer tomography technique. In this way, it is possible to track the penetration of the ADC administered to the subject into the tumor tissue and the like.

### <Anti-Cancer Composition>

As shown in Examples described later, the ADC of the present invention exhibits a significant tumor growth inhibitory effect in vivo, and can also exhibit a tumor regression effect. Therefore, the present invention provides a composition (anticancer composition) for treating cancer, containing the ADC of the present invention.

Examples of the "cancer" as the subject of the present invention include solid tumors, and more specific examples include pancreatic cancer, lymphoma, lung cancer, head and neck cancer, prostate cancer, bladder cancer, breast cancer, esophageal cancer, stomach cancer, colon cancer, uterine cancer, ovarian cancer, skin cancer, thyroid cancer, thoracic cancer, kidney cancer, testis cancer, penile cancer, liver cancer, biliary tract cancer, brain tumor, bone and soft tissue tumor, retroperitoneal tumor, angiosarcoma, and lymphangiosarcoma. In addition, such cancers may be primary or metastatic.

In addition to ADCs, the compositions of the present invention can include additional pharmacologically acceptable components. Examples of these additional components include supports, emulsifiers, wetting agents, pH buffering agents, excipients, disintegrants, buffer agents, isotonic agents, suspensions, solubilizers, soothing agents, stabilizers, preservatives, and antiseptics. More specifically, in the case of liquid preparations such as injections, examples of the pharmacologically acceptable additional components include aqueous solutions (such as physiological saline, water for injection, phosphate buffer solution, glucose aqueous solution, and glycerol aqueous solution), aluminum hydroxide, and the like. Further, in the case of freeze-dried preparations, examples include, but not limited to, sugar (such as mannitol, lactose, and saccharose), albumin, and the like. Further, the compositions of the present invention may be in the form of a kit such that the above components can be mixed before administration. In addition, when used as injections, they may be in the form introduced into a syringe.

The compositions of the present invention may include only the ADC of the present invention as an active ingredient, or may include the ADC and at least one additional cancer therapeutic agent. The ADC of the present invention can also be administered together with an additional cancer therapeutic agent, thereby enhancing the anticancer effect. The additional anticancer agent used for such purposes may be administered to the subject simultaneously with, separately from, or continuously with the ADC of the present invention, or may be administered at different dosing intervals. Examples of such cancer therapeutic agent include immune checkpoint inhibitors (such as anti-PD-1 antibody), gemcitabine, S-1, erlotinib, 5-FU, leucovorin, irinotecan (CPT-11), oxaliplatin, Abraxane, carboplatin, paclitaxel, pemetrexed, sorafenib, vinblastine, LH-RH analogs (such as leuprorelin and goserelin), estramustine phosphate, estrogen antagonists (such as tamoxifen and raloxifene), aromatase inhibitors (such as anastrozole, letrozole, and exemestane), and the drugs described in International Publication No. WO2003/038043, but are not limited as long as they are drugs with antitumor activity.

### <Cancer Treatment Method>

The present invention also provides a method for treating cancer, including administering an effective amount of the ADC of the present invention or a composition containing the ADC to a subject.

The ADCs and compositions administered in this way are as described above. The "subject" to which they are administered is not particularly limited as long as it requires treatment, and may be one that has the above-mentioned cancer, one that may have the cancer, or one that may have a recurrence of the cancer. In addition, the subject may be a human or a non-human mammal. The non-human mammal is not particularly limited, and examples thereof include livestock, pets, and laboratory animals, and more specific examples include monkeys, mice, rats, hamsters, guinea pigs, cows, pigs, horses, rabbits, sheep, goats, cats, and dogs.

In the present invention, "treatment" means complete cure, recovery, remission, alleviation of symptoms or conditions, and decrease in progression (worsening) rate. Further, the "effective amount" means the amount of the drug effective for the treatment.

The method for administering the ADC or composition of the present invention differs depending on the age, body weight, gender, health condition, and the like of the administration subject, and can be administered by any of parenteral administration (such as intravenous administration, intraarterial administration, intradermal administration, intramuscular administration, intraperitoneal administration, or local administration) or oral administration. A preferable method for administration is parenteral administration, more preferably intravenous administration. Administration can be, for example, by injection or bolus injection.

The dose of the ADC or composition of the present invention may vary depending on the age, weight, gender, health condition, degree of progression of symptoms, and components of the composition to be administered. Generally, in the case of intravenous administration, it is 0.001 to 1000 mg, preferably 0.01 to 100 mg, per kg of body weight per day for an adult in terms of the amount of the drug contained. The number of administrations may be, for example, once, or multiple times at intervals of 1 day to 1 year (for example, every week, every 10 to 30 days, every month, every 3 to 6 months, every 6 months, and every year) .

### [Examples]

Hereinafter, the present invention is described in more detail based on Examples, but the present invention is not limited to the following Examples.

### [Preparation of Anti-Tissue Factor Antibody]

In this Example, a monoclonal antibody (rat 1084 antibody) produced from the rat-derived hybridoma clone No. 1084 described in PTL 2 was humanized and used as an anti-tissue factor antibody.

Specifically, first, the sequence of each CDR region of the rat 1084 antibody was determined by the definition of Kabat. Each of the determined sequences of the rat 1084 antibody is as follows:
heavy chain variable region, amino acid sequences of CDR1 to 3 ... SEQ ID NOs: 9 to 11
amino acid sequence of heavy chain region ... SEQ ID No: 12 light chain variable region, amino acid sequences of CDR1 to 3 ... SEQ ID NOs: 13 to 15
amino acid sequence of light chain region ... SEQ ID No: 16.

Next, the framework region of the rat 1084 antibody and the like were substituted with those derived from humans according to the method described in Chapter 7 (123-138) of Human Monoclonal Antibody (Springer Protocols). Each amino acid sequence of the humanized 1084 antibody is as follows:
heavy chain variable region, amino acid sequences of CDR1 to 3 ... SEQ ID NOs: 1 to 3
amino acid sequence of heavy chain region ... SEQ ID No: 4
light chain variable region, amino acid sequences of CDR1 to 3 ... SEQ ID NOs: 5 to 7
amino acid sequence of light chain region ... SEQ ID No: 8.

Note that the sequences of some CDR regions are different between the humanized 1084 antibody and the rat 1084 antibody. This is because the human sequence was employed instead of the rat sequence, as these parts are not exposed on the surface of the molecule and interact with the humanized framework region, rather than the antigen recognition site, to contribute to structural stabilization.

Further, the sequence other than the variable region (constant region) used was that of rituximab (monoclonal antibody composed of anti-human CD20 human/mouse chimeric antibody, CAS Registry Number: 174722-31-7, DrugBank: DB00073). The amino acid sequences of the heavy and light chains of the humanized 1084 antibody with the sequences derived from rituximab are set forth in SEQ ID NOs: 17 and 18, respectively.

Then, a vector encoding the humanized 1084 antibody was prepared by a conventional method, and CHO cells (trade name; ExpiCHO expression system (manufactured by Thermo Fisher)) were used to transiently express and purify the antibody.

### [Preparation of Antibody Drug Conjugates (ADCs)]

The humanized 1084 antibody prepared above was used to prepare four types of ADCs shown in Table 1 below by the method shown below.

**[Table 1]**

| | Antibody | Linker | Cytotoxic Agent | Drug-to-Antibody Ratio (DAR) |
|---|---|---|---|---|
| hu1084-Dxd3 | Humanized 1084 Antibody | MC-Gly-Gly-Phe-Gly | Dxd | 3 |
| hu1084-Dxd8 | Humanized 1084 Antibody | MC-Gly-Gly-Phe-Gly | Dxd | 8 |
| hu1084-PEG12-MMAE3 | Humanized 1084 Antibody | Mal-PEG12-Val-Cit-PABA | MMAE | 3 |
| hu1084-PEG12-MMAE8 | Humanized 1084 Antibody | Mal-PEG12-Val-Cit-PABA | MMAE | 8 |
| hu1084-MMAE3 | Humanized 1084 Antibody | Mal-Val-Cit-PABA | MMAE | 3 |
| hu1084-MMAE8 | Humanized 1084 Antibody | Mal-Val-Cit-PABA | MMAE | 8 |

### (Preparation of hu1084-Dxd3)

First, a buffer used during the reduction treatment was prepared. As buffer A, Dulbecco's phosphate-buffered saline (DPBS) containing 5 mM EDTA (pH 8.0) was prepared. Further, as buffer B, 100 mM sodium dihydrogen phosphate, 150 mM sodium chloride, and 5 mM EDTA (pH 8.0) were prepared.

Next, 0.5 M EDTA (pH 8.0) was added to the DPBS solution containing the antibody to adjust the final EDTA concentration to 5 mM. Then, the amount of solution with the final antibody concentration equal to 1 mg/mL was determined as the total reaction solution amount, and buffer B in 1/6 of that amount was mixed with the antibody solution, and finally buffer A was used to adjust the final antibody concentration to 1 mg/mL.

This reduction reaction mixture solution was preincubated at 37°C for 15 minutes in a constant temperature bath. Then, the reducing agent in 1/100 amount of the whole mixture solution (2-mercaptoethylamine-HCl solution, manufactured by Sigma-Aldrich, final concentration 20 mM) was added, and the mixture was incubated at 37°C for 30 minutes using a constant temperature bath.

After completion of the reaction, the sample was immediately transferred onto ice, and buffer substitution was performed on buffer A using VIVASPIN TURBO 15 (manufactured by Sartorius, Gottingen, Germany) to remove the reducing agent. The buffer substitution was repeated until the original reaction solution could be diluted 1 million times or more.

Subsequently, the amount of solution with the final antibody solution concentration equal to 0.5 mg/mL was determined as the total reaction solution amount, and buffer B in 1/6 of that amount was mixed with an antibody solution containing the reduced antibody and with the linker Dxd in a molar amount three times the required amount calculated from the free thiol groups (MedChemExpress, product number: HY-13631E, trade name: Deruxtecan), and buffer A was used to adjust the final antibody concentration to 0.5 mg/mL. The mixture solution was gently stirred by inversion and mixing, and then allowed to stand at 4°C for 18 hours.

Note that the linker Dxd is a compound represented by the following formula.

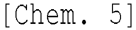

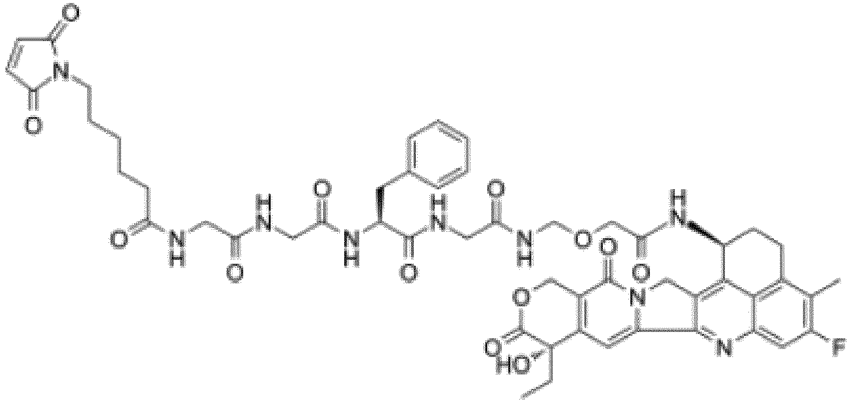

Finally, VIVASPIN TURBO 15 was used again to perform buffer substitution on DPBS. This buffer substitution process was also repeated until the original reaction solution could be diluted 1 million times or more.

The ADC thus prepared was filtered using a PVDF membrane 0.22 um filter unit under sterile conditions, and then dispensed in sterile DPBS to an antibody concentration of 2 mg/mL and cryopreserved at -80°C until use. Freezing was performed only once, and it was used for each experiment immediately after thawing from freeze.

### (Preparation of hu1084-Dxd8)

An ADC was prepared in the same manner as described above (Preparation of hu1084-Dxd3) except that the temperature of the pre-incubation of the reduction reaction mixture solution was set to 26°C, the reducing agent added to the mixture solution was used as a DL-DTT solution (manufactured by Sigma-Aldrich, final concentration 35 mM), and the incubation temperature after the addition was set to 26°C.

### [Preparation of hu1084-PEG12-MMAE3]

An ADC was prepared in the same manner as described above (Preparation of hu1084-Dxd3) except that the following linker PEG12-MMAE (provided by Mal-PEG12-vc-PABC-MMAE, Professor Shino Manabe, RIKEN) was used instead of the linker Dxd. Note that the linker PEG12-MMAE is a compound represented by the following formula.

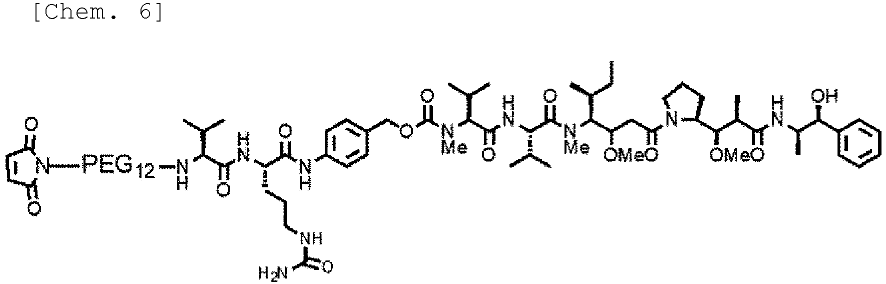

### [Preparation of hu1084-PEG12-MMAE8]

An ADC was prepared in the same manner as described above (Preparation of hu1084-Dxd8) except that the linker PEG12-MMAE was used instead of the linker Dxd.

### [Preparation of hu1084-MMAE3]

An ADC was prepared in the same manner as described above (Preparation of hu1084-Dxd3) except that the following linker MMAE (Mal-vc-PABC-MMAE, trade name: vcMMAE, manufacturer: MedChemExpress, model number: HY-15575) was used instead of the linker Dxd. Note that the linker MMAE is a compound represented by the following formula.

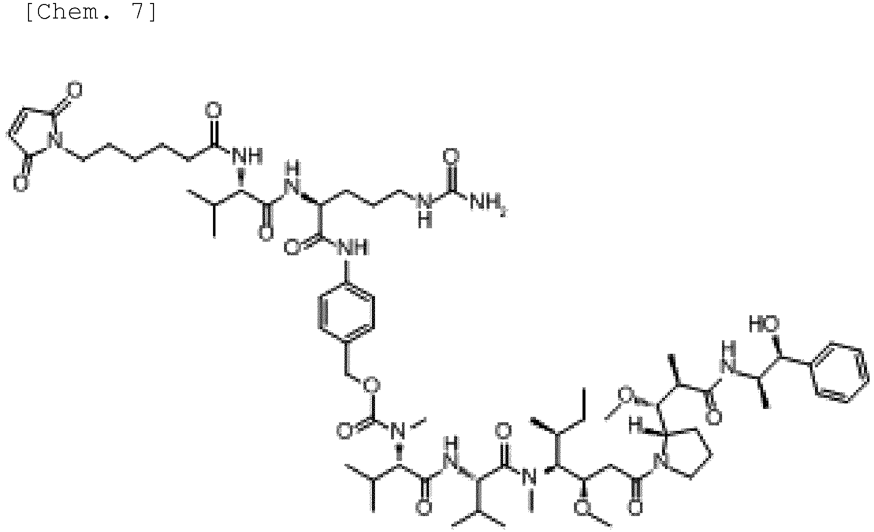

### [Preparation of hu1084-MMAE8]

An ADC was prepared in the same manner as described above (Preparation of hu1084-Dxd8) except that the linker MMAE was used instead of the linker Dxd.

The average number of the linkers and drugs bonded per antibody (DAR) in these ADCs was measured using DTNB (5,5'-dithiobis(2-nitrobenzoic acid)), a reagent for colorimetrically quantifying thiol groups. That is, the reagent was added after the reduction of the antibody with a reducing agent and after the addition of the linker compound. Then, the free thiol groups were quantified by measuring the absorbance (λmax = 412 nm). Then, the DAR was calculated based on the number of free thiol groups after the reduction and after the addition.

### [Cell-Killing Effect]

The ADCs prepared above were evaluated in vitro for their cell-killing effects on human pancreatic adenocarcinoma cells by the following method.

RPMI-1640 supplemented with 10% fetal bovine serum and penicillin-streptomycin-ampicillin B (× 100) suspension was used as the culture medium. Human pancreatic adenocarcinoma BxPC-3, PSN-1, and HPAF-II were adjusted to 5000 cells/mL, 2000 cells/mL, and 5000 cells/mL, respectively, and seeded on 96-well plates at 100 pL/well and allowed to stand overnight in a CO₂ incubator (37°C).

The ADCs, Dxd single agent, and MMAE single agent were each adjusted with a culture solution so that the final concentration (in terms of MMAE and Dxd) was 0.1, 0.3, 1, 3, 10, 30, 100 nM, or the like, and were added at 100 µL/well to a 96-well plate inoculated with each human pancreatic adenocarcinoma, and then allowed to stand in a CO₂ incubator (37°C) for 6 days. Then, the culture medium containing the drug was removed with an aspirator, and the number of viable cells was measured by Cell Counting Kit-8 (manufactured by Dojindo Laboratories) according to the attached protocol. The reaction time of the reagent was three hours, and the absorbance was measured by SpectraMax Paradigm (manufactured by Molecular Devices). Figs. 1A to 1E show the results of the cell-killing effects.

From the results shown in Fig. 1A, the IC50 values of MMAE, hu1084-PEG12-MMAE3, hu1084-PEG12-MMAE8, Dxd, hu1084-Dxd3, and hu1084-Dxd8 for BxPC-3 were, respectively, 0.62 ± 0.04 nM, 0.05 ± 0.00 nM, 0.05 ± 0.00 nM, 2.58 ± 0.26 nM, 0.27 ± 0.02 nM, and 0.20 ± 0.01 nM.

From the results shown in Fig. 1B, the IC50 values of MMAE, hu1084-PEG12-MMAE3, hu1084-PEG12-MMAE8, Dxd, hu1084-Dxd3, and hu1084-Dxd8 for PSN-1 were, respectively, 0.73 ± 0.04 nM, 6.29 ± 1.04 nM, 0.35 ± 0.12 nM, 7.92 ± 0.84 nM, N.D., and N.D.

In addition, from the results shown in Figs. 1C to 1F, the IC50 value of free MMAE was 30 to 50 times lower than that of free Dxd in the 3 types of human pancreatic adenocarcinoma cell lines. That is, the cell-killing effect on these human pancreatic adenocarcinoma cell lines was significantly higher in MMAE. In addition, MMAE had a several-fold higher cell-killing effect in each ADC containing these drugs.

As described above, in any human pancreatic adenocarcinoma cell, the conjugates composed of the MMAE linker (linker PEG12-MMAE or linker MMAE) and the anti-tissue factor antibody showed a higher cell-killing effect than the conjugates composed of the linker Dxd and the anti-tissue factor antibody. In particular, for PSN-1, hu1084-Dxd3 and hu1084-Dxd8 did not give an IC50 (both are N.D.).

### [Antitumor Effect]

### (Preparation of Patient-Derived Xenograft (PDX) Model)

Next, for each ADC, the antitumor effect on human pancreatic cancer cells was evaluated in vivo by the following method.

In all experiments, 5-week-old nude mice (BALB/c nu/nu, female, Charles River Laboratories Japan, Inc.) acclimatized for 1 week were used. Human pancreatic tumor tissue fragments of 3 to 5 mm square (model number: PAN-02-JCK, PAN-04-JCK, PAN-08-JCK, PAN-14-JCK (Central Institute for Experimental Animals)) were transplanted subcutaneously to the nude mice, and the formed tumor was designated as Passage 1 (P1). Note that each human pancreatic tumor tissue (human suffering from each pancreatic tumor) is of different origin. The P1 tumor was shredded, trimmed to 3-5 mm squares, and transplanted subcutaneously into mice to obtain a P2 tumor. Tumor passage was performed by repeating this operation. It was decided to use P4 to P6 for the examination of antitumor effects.

### (Immunohistochemical Staining)

After euthanizing the mice by over-anesthesia, the tumor tissues were rapidly removed, embedded in OCT Compound and cryopreserved at -80°C. Tumor tissues with a length of 6 um were sliced using a cryostat, each attached to a glass slide, and air-dried for 30 minutes. They were fixed with a 4% paraformaldehyde (PFA)-DPBS solution at room temperature for 15 minutes and washed with DPBS, and then treated with a 3% hydrogen peroxide solution-methanol solution at room temperature for 30 minutes to inactivate the endogenous peroxidase. Then, the tissues were washed with DPBS and blocked with 5% skim milk-DPBS solution for 1 hour at room temperature. Similarly, they were washed with DPBS, and a goat-derived anti-tissue factor polyclonal antibody (R&D Systems, Inc.) was diluted 1/500 with a 5% skim milk solution and reacted at room temperature for 1 hour. After washed with DPBS, HRP-labeled anti-goat polyclonal antibody (manufactured by MBL) was diluted 1/500 with 5% skim milk solution and reacted at room temperature for 1 hour. After washed with DPBS, diaminobenzidine (DAB) was reacted at room temperature for 3 minutes, washed again with DPBS, and nuclear staining with hematoxylin solution was performed at room temperature for 10 minutes. Coloring was performed with running water for 10 minutes, and dehydration and clearing were performed with ethanol and xylene. Finally, using Mount-Quick, the tissue was sealed by covering it with a cover glass. Observation was performed using Virtual Slide System (manufactured by Olympus). Figs. 2A to 2C, 3D, and 3E show the results of staining.

As shown in Figs. 2A to 2C, the expression of tissue factor was confirmed in 3 cases of pancreatic cancer PDX models (no, 2, 4, and 8). In any of these models, some tissue factor-negative cancer cell populations were also found, and the tumors were heterogeneous in terms of tissue factor expression. Further, as shown in Fig. 3E, the tumors in no, 14 were also heterogeneous in terms of tissue factor expression, as in the above three cases. In particular, the pancreatic cancer PDX models no, 8 and no, 14 had lower tissue factor expression levels than no, 2 and 4, and in their expressions, the tumors were also more heterogeneous, with 50% or more of all cancers expressing no tissue factor.

### (Treatment Experiment Using PDX Model)

When the average tumor size of the PDX models reached 200 to 300 mm³, mice were randomly divided into groups, and treatment experiments were started by tail vein administration in each administration group. Each ADC was administered at 10 mg/kg once a week for a total of 3 times, and tumor size and body weight were measured once a week. The tumor size was calculated by measuring the major axis and minor axis of the subcutaneous tumor using an electric caliper and calculating by "tumor major axis × minor axis × minor axis × 1/2". The time when the tumor size exceeded 2000 mm³ or the time when an ulcer or the like was observed in the tumor part was determined as a humane endpoint, and the mice were euthanized by over-anesthesia with isoflurane. The number of N was 3 or 4. In addition, as a negative control group, a mouse treated with DPBS instead of ADC was also prepared.

As shown in Figs. 3A to 3C, in all the PDX models, a tumor regression or tumor growth inhibitory effect was observed when administered with conjugates composed of the linker Dxd and an anti-tissue factor antibody, contrary to the above-mentioned in vitro results.

Meanwhile, in the case of administering conjugates composed of an MMAE linker (linker PEG12-MMAE or linker MMAE) and an anti-tissue factor antibody, a tumor growth inhibitory effect was observed when hu1084-PEG12-MMAE8 was administered to the PDX model no. 8 transplanted with the human pancreatic tumor tissue fragment PAN-08-JCK, as shown in Fig. 3C. In addition, a tumor growth inhibitory effect was observed when hu1084-MMAE3 was administered to the PDX model no. 14 transplanted with the human pancreatic tumor tissue fragment PAN-14JCK, as shown in Fig. 3F. However, the degree of those effects was inferior to that when the conjugate composed of the linker Dxd and the anti-tissue factor antibody was administered.

As described above, it has been clarified that the ADC composed of the anti-tissue factor antibody and the linker Dxd can exert a high tumor regression or tumor growth inhibitory effect on cancer in vivo. Particularly surprisingly, a cancer cell population negative for some tissue factor was also observed, and even if the tumor was heterogeneous in terms of tissue factor expression, the ADC showed a high tumor growth inhibitory effect and the like.

It is not exactly clear why such an effect can be obtained, but the present inventors infer as follows. Specifically, Dxd is highly hydrophobic, and even after attacking tissue factor-positive cancer cells as ADC, it is released extracellularly in a free state and passes through the cell membranes of adjacent tissue factor-negative cancer cells. Then, it can easily invade the cells and kill the cancer cells (bystander effect). Most of the solid cancers formed in the body are heterologous cancer tissues in the expression of tissue factor, strongly suggesting the effectiveness of ADC composed of anti-tissue factor antibody and linker Dxd.

Note that although not shown in the figures, when the linker MMAE was used, unlike the linker PEG12-MMAE and the linker Dxd shown in Figs. 3A to 3C, the tumor growth inhibitory effect was higher when the DAR was 3 than when it was 8. In addition, no significant weight loss was observed in the ADC-administered group in all treatment experiments.

### [Surface Plasmon Resonance Analysis]

The dissociation constant and association constant of the humanized 1084 antibody were analyzed by surface plasmon resonance (SPR, trade name: BiaCore T200 (manufactured by GE healthcare)).

Specifically, first, 1 mg/mL recombinant human tissue factor antigen was added to 10 mM sodium acetate solution (pH 5.0) to adjust the final concentration to 20 µg/mL. Next, the contact time was set to 120 seconds, and the antigen was immobilized by the amine coupling method on a CM5 sensor chip (manufactured by GE healthcare). On the other hand, the humanized 1084 antibody was solution-substituted with an HBS-N solution (manufactured by GE healthcare), and the antibody concentrations were adjusted to 320 nM, 160 nM, 80 nM, 40 nM, and 20 nM. Then, these diluted antibodies were used to measure the binding kinetics of the antibodies by single-cycle kinetics. The antibody contact time and dissociation measurement time were set to 120 seconds and 600 seconds, respectively. The analysis of the sensorgram was performed by 1 : 1 binding. Table 2 shows the obtained results.

**[Table 2]**

| | kₐ(1/Ms) | k_{d}(1/s) | K_{D}(M) |
|---|---|---|---|
| hu1084 mAb | 6.573 × 10⁴ | 1.788 × 10⁻³ | 2.719 × 10⁻⁸ |

### [Industrial Applicability]

As described above, the present invention makes it possible to provide an antibody drug conjugate containing an anti-tissue factor antibody and a cytotoxic agent, which exerts an antitumor effect in vivo. Therefore, the present invention is useful in the development of anticancer agents, the treatment of cancer, and the like.

## Claims

1. A conjugate in which an antibody that binds to tissue factor is bound to a linker and drug represented by the following formula.

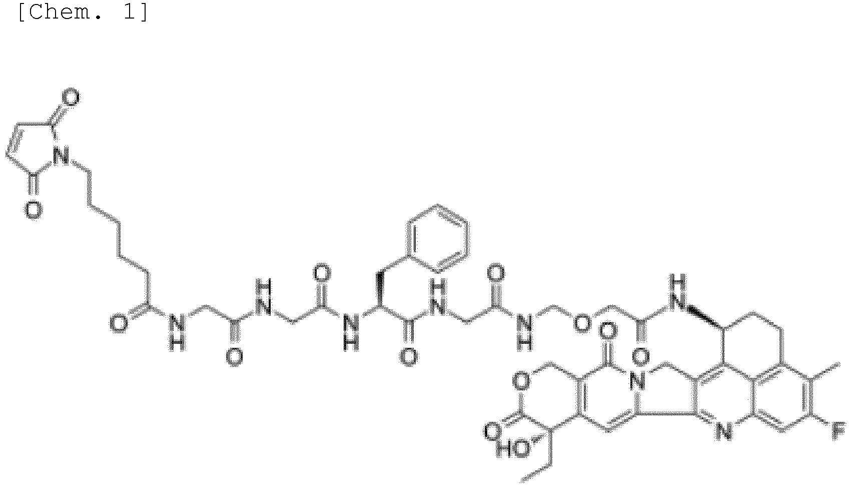

2. The conjugate according to claim 1, wherein the antibody that binds to tissue factor has a dissociation rate constant kd of 1 × 10⁻⁴ (1/s) or more and an association rate constant Ka of 1 × 10⁴ (1/Ms) or more.

3. The conjugate according to claim 1 or 2, wherein the antibody that binds to tissue factor is an antibody that comprises
a heavy chain variable region containing, as CDRs 1 to 3, the amino acid sequences of SEQ ID NOs: 1 to 3, or the amino acid sequences of SEQ ID NOs: 1 to 3 in at least any one of which one or more amino acids are substituted, deleted, added, and/or inserted and
a light chain variable region containing, as CDRs 1 to 3, the amino acid sequences of SEQ ID NOs: 5 to 7, or the amino acid sequences of SEQ ID NOs: 5 to 7 in at least any one of which one or more amino acids are substituted, deleted, added, and/or inserted.

4. The conjugate according to claim 1 or 2, wherein the antibody that binds to tissue factor is an antibody that comprises
a heavy chain variable region containing the amino acid sequence of SEQ ID NO: 4, the amino acid sequence having a homology of 80% or more with the amino acid sequence of SEQ ID NO: 4, or the amino acid sequence of SEQ ID NO: 4 in at least any portion of which one or more amino acids are substituted, deleted, added, and/or inserted and
a light chain variable region containing the amino acid sequence of SEQ ID NO: 8, the amino acid sequence having a homology of 80% or more with the amino acid sequence of SEQ ID NO: 8, or the amino acid sequence of SEQ ID NO: 8 in at least any portion of which one or more amino acids are substituted, deleted, added, and/or inserted.

5. The conjugate according to any one of claims 1 to 4, wherein an average number of linkers and drugs bonded per antibody is 3 to 8.

6. An anti-cancer composition comprising: the conjugate according to any one of claims 1 to 5.

7. An anti-pancreatic cancer composition comprising: the conjugate according to any one of claims 1 to 5.
